(19) Europäisches Patentamt
European Patent Office
Office européen des brevets

(11) EP 1 375 501 B1

(12) EUROPÄISCHE PATENTSCHRIFT

(45) Veröffentlichungstag und Bekanntmachung des Hinweises auf die Patenterteilung:
**02.11.2005 Patentblatt 2005/44**

(51) Int Cl.[7]: **C07D 471/04**, C07B 57/00

(21) Anmeldenummer: **03012664.3**

(22) Anmeldetag: **04.06.2003**

(54) **Verfahren zur Enantiomerenanreicherung von cis-8-Benzyl-7,9-dioxo-2,8-diazabicyclo(4.3.0)nonan unter Verwendung von Gegenstromchromatographie**

Process for enantiomeric enrichment of cis-8-benzyl-7,9-dioxo-2,8-diazabicyclo(4.3.0)nonane using simulated moving bed chromatography

Enrichissement d'enantiomères du cis-8-benzyl-7,9-dioxo-2,8-diazabicyclo(4.3.0)nonane en utilisant la chromatographie au lit mobile simulé

(84) Benannte Vertragsstaaten:
**AT BE BG CH CY CZ DE DK EE ES FI FR GB GR HU IE IT LI LU MC NL PT RO SE SI SK TR**

(30) Priorität: **17.06.2002 DE 10226923**

(43) Veröffentlichungstag der Anmeldung:
**02.01.2004 Patentblatt 2004/01**

(73) Patentinhaber: **LANXESS Deutschland GmbH**
**51369 Leverkusen (DE)**

(72) Erfinder:
- **Diehl, Herbert, Dr.**
  **51373 Leverkusen (DE)**
- **Krebs, Andreas, Dr.**
  **verstorben (DE)**
- **Lange, Walter, Dr.**
  **51519 Odenthal (DE)**
- **Paul, Hanns-Ingolf, Dr.**
  **51375 Leverkusen (DE)**
- **Seidel, Dietrich, Dr.**
  **42111 Wuppertal (DE)**
- **Grosser, Rolf, Dr.**
  **51373 Leverkusen (DE)**
- **Reichelt, Tobias, Dr.**
  **51065 Köln (DE)**

(56) Entgegenhaltungen:
**EP-A- 0 550 903**
**EP-A- 1 067 129**
**WO-A-00/76996**
**WO-A-92/16274**

- **SCHULTE M ET AL: "Preparative enantioseparation by simulated moving bed chromatography" JOURNAL OF CHROMATOGRAPHY, ELSEVIER SCIENCE PUBLISHERS B.V. AMSTERDAM, NL, Bd. 906, Nr. 1-2, 12. Januar 2001 (2001-01-12), Seiten 399-416, XP004227671 ISSN: 0021-9673**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 375 501 B1

## Beschreibung

Die vorliegende Erfindung betrifft ein Verfahren zur Enantiomerenanreicherung von cis-8-Benzyl-7,9-dioxo-2,8-diazabicyclo[4.3.0]nonan mit Hilfe von kontinuierlicher Gegenstromchromatographie, wie insbesondere der SMB-Chromatographie (SMB = Simulated Moving Bed) .

In einem weiteren Aspekt betrifft die Erfindung ein Verfahren zur Herstellung von (1S,6R)-8-Benzyl-7,9-dioxo-2,8-diazabicyclo[4.3.0]nonan unter Verwendung des zuvor genannten Verfahrens, das weiterhin einen Racemisierungsschritt einschließt.

Die Enantiomeren des cis-8-Benzyl-7,9-dioxo-2,8-diazabicyclo[4.3.0]nonans (DOPP) sind wertvolle Intermediate zur Herstellung von Chinolon- und Naphthyridoncarbonsäurederivaten, die unter anderem als wirksamer Bestandteil antibakterieller Mittel und Futterzusatzstoffe große technische Bedeutung gewonnen haben (EP-A 550 903).

(1S,6R)-8-Benzyl-7,9-dioxo-2,8-diazabicyclo[4.3.0]nonan der Formel (Ia)

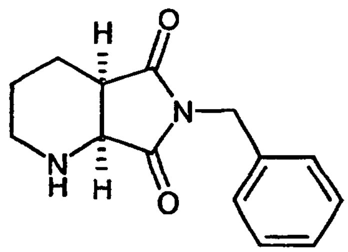

(Ia)

stellt beispielsweise ein wertvolles Zwischenprodukt für die Herstellung des (S,S)-2,8-diazabicyclo[4.3.0]nonans (II) dar,

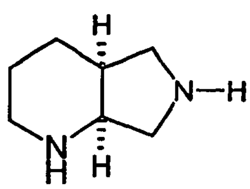

(IIa),

in das es durch Reduktion der Carbonylgruppen und Debenzylierung in an sich bekannter Weise (EP-A 350 733) überführt werden kann. Das (S,S)-2,8-Diazabicyclo[4.3.0]nonan wird seinerseits für die Herstellung des Antibiotikums Moxifloxacin (INN, 1-Cyclopropyl-7-([S,S]-2,8-diazabicyclo[4.3.0]non-8-yl)-6-fluor-1,4-dihydro-8-methoxy-4-oxo-3-chinoloncarbonsäure, (III) verwendet (EP-A 350 733):

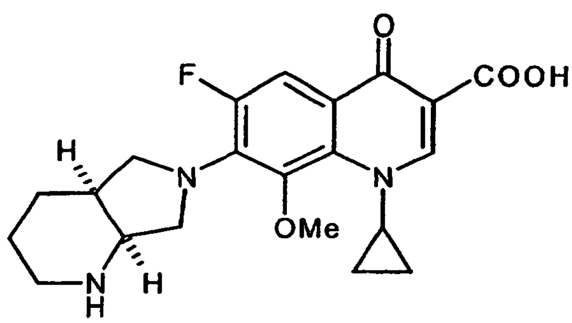

(III)

Das Enantiomere (1R,6S)-8-Benzyl-7,9-dioxo-2,8-diazabicyclo[4.3.0]nonan (Ib)

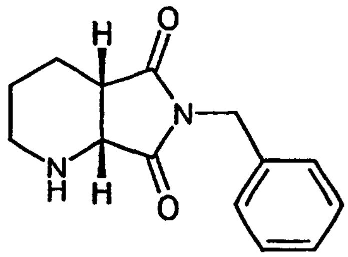

(Ib)

stellt wiederum ein wertvolles Zwischenprodukt für die Herstellung des (R,R)-2,8-diazabicyclo[4.3.0]nonans (IIb) dar,

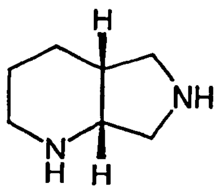

(IIb)

das ebenfalls für die Herstellung sehr wirksamer antibakterieller Mittel verwendet werden kann (z.B. Interscience Conference on Antimicrobial Agents and Chemotherapy (ICAAC), 1996, Abstr. No. F-001).

Verfahren zur Enantiomerenanreicherung von cis-8-Benzyl-7,9-dioxo-2,8-diazabicyclo[4.3.0]nonan sind prinzipiell bekannt.

So ist beispielsweise aus der EP-A 550 903 ein Verfahren zur Racematspaltung des cis-8-Benzyl-7,9-dioxo-2,8-diazabicyclo[4.3.0]nonans unter Verwendung von Weinsäure bekannt (Beispiel A, Methode IV sowie Beispiel B, Methode II a)). Die dort beschriebenen Verfahren erfordern für die Herstellung des (1S,6R)-Enantiomers mehrmaliges Umkristallisieren der diastereomeren D-(-)-Weinsäuresalze bzw. die Umsetzung mit L-(+)-Weinsäure sowie nachfolgende Reaktion der freigesetzten Mutterlauge mit D-(-)-Weinsäure und Umkristallisation. Die erhaltenen Enantiomerenüberschüsse sind mit 93,8 % ee für das (1R,6S)-Enantiomer und 96,6 % ee für das (1S,6R)-Enantiomer im Hinblick auf die Vielzahl der Operationen und die große Menge an chiralen Hilfsreagentien unzureichend und somit für den technischen Einsatz nur bedingt geeignet.

Aus EP-A 1 192 153 ist ein Verfahren zur Racematspaltung des cis-8-Benzyl-7,9-dioxo-2,8-diazabicyclo [4.3.0]nonans bekannt, das unter anderem (-)-2,3:4,6-Di-O-isopropyliden-2-keto-L-gulonsäure und Camphersulfonsäure als chirale Hilfsreagenzien einsetzt. Aber auch hier begrenzt die benötigte Menge des chiralen Hilfsreagenzes und die Vielzahl der Arbeitsschritte den technischen Einsatz.

Es bestand daher das Bedürfnis nach einem effizienten Verfahren zur Enantiomerenanreicherung von cis-8-Benzyl-7,9-dioxo-2,8-diazabicyclo[4.3.0]nonan, wobei die getrennten Enantiomere im industriellen Maßstab und hoher absoluter und optischer Reinheit bereitgestellt werden können.

Es wurde nun überraschenderweise ein Verfahren zur Enantiomerenanreicherung von cis-8-Benzyl-7,9-dioxo-2,8-diazabicyclo[4.3.0]nonan gefunden, das dadurch gekennzeichnet ist, dass die Enantiomerenanreicherung durch kontinuierliche Gegenstromchromatographie erfolgt.

Das Verfahren der kontinuierlichen Gegenstromchromatographie ist aus der Literatur bekannt (siehe etwa M. Schulte, J. Strube, Journal of Chromatography A, 906 (2001), 399-416).

Der Begriff "Enantiomerenanreicherung" ist im Rahmen der Erfindung so zu verstehen, dass ein Ausgangsgemisch, das die beiden Enantiomeren von cis-8-Benzyl-7,9-dioxo-2,8-diazabicyclo[4.3.0]nonan, (1S,6R)- und (1R, 6S)-8-Benzyl-7,9-dioxo-2,8-diazabicyclo[4.3.0]nonan enthält, in der Weise getrennt wird, dass nach der Trennung die Enantiomere in höheren optischer Reinheit vorliegen als vor der Trennung.

Es sei darauf hingewiesen, dass im Rahmen der Erfindung die aufgeführten allgemeinen oder in Vorzugsbereichen aufgeführten Definitionen, Parameter und Erläuterungen untereinander, also auch zwischen den jeweiligen Bereichen und Vorzugsbereichen, beliebig kombiniert werden können und auch diese Kombinationen vom Umfang der Erfindung umfasst sind.

Für das erfindungsgemäße Verfahren wird bevorzugt ein Ausgangsgemisch eingesetzt, dass die Enantiomeren in einem molaren Verhältnis von 0,25:1 bis 4:1 und bevorzugt 0,8:1 bis 1,25:1 enthält. Besonders bevorzugt enthält

das Ausgangsgemisch die racemische Mischung der Enantiomeren.

Die optische Reinheit wird im Folgenden durch den "enantiomeric excess" (ee) angegeben, der definiert ist als: .

$$ee [S] = (m[S] - m[R])/m(S+R)$$

wobei

ee(S) die optische Reinheit des Enantiomers S, m(S) die Stoffmenge des Enantiomers S und m(R) die Stoffmenge des Enantiomers R ist. Die Angabe erfolgt üblicherweise in Prozent enantiomeric excess (% ee = ee/100).

Cis-8-Benzyl-7,9-dioxo-2,8-diazabicyclo[4.3.0]nonan kann als racemisches Gemisch gemäß EP-A 350 733 vorteilhaft durch Kernhydrierung von Pyridin-2,3-dicarbonsäure-N-benzylimid erhalten werden. Bei dieser Herstellungsmethode können prinzipiell auch die zu den cis-Verbindungen diastereomeren trans-Verbindungen als Nebenprodukte anfallen. Weiterhin können auch andere organische Nebenprodukte erzeugt werden. Es wurde überraschenderweise gefunden, dass die erfindungsgemäße Enantiomerenanreicherung auch in Gegenwart dieser Nebenprodukte durchgeführt werden kann.

Die Erfindung umfasst daher auch ein Verfahren, in dem die Enantiomerenanreicherung von cis-8-Benzyl-7,9-dioxo-2,8-diazabicyclo[4.3.0]nonan in Gegenwart der enantiomeren trans-8-Benzyl-7,9-dioxo-2,8-diazabicyclo [4.3.0]nonane und/oder anderen aus der Kernhydrierung von Pyridin-2,3-dicarbonsäure-N-benzylimid stammenden organischen Nebenprodukten durchgeführt wird.

Deren Masse-Gehalt kann bezogen auf das für das erfindungsgemäße Verfahren eingesetzte Ausgangsgemisch, das die Enantiomeren des cis-8-Benzyl-7,9-dioxo-2,8-diazabicyclo[4.3.0]nonans enthält, beispielsweise 0,01 bis 20 % betragen, üblicherweise 0,5 bis 10 %.

Das Prinzip der kontinuierlichen Gegenstromchromatographie zur Trennung chiraler Verbindungen ist zum Beispiel aus M. Negawa und F. Shoji, J. Chrom. 590, 1992, Seiten 113-117 bekannt. Geeignete Anlagen zur Durchführung der kontinuierlichen Gegenstromchromatographie wie insbesondere SMB-Anlagen sind beispielsweise beschrieben in US 2,621,149; US 2,985,589 sowie WO 92/16274 und kommerziell erhältlich.

Dabei wird im Allgemeinen in einer SMB-Anlage durch zwei oder mehr miteinander verbundene Segmente ein sich in einer Richtung bewegender und gegebenenfalls zirkulierender Flüssigkeitsstrom erzeugt, wobei jedes Segment über mindestens eine mit einer chiralen stationären Phase befüllte Säule verfügt und in Strömungsrichtung zumindest mit einem Flüssigkeitseinlass und einem Flüssigkeitsauslass versehen ist und wobei jedes Segment über zumindest einen Einlass verfügt, über den dem gegebenenfalls zirkulierenden Flüssigkeitsstrom ein Feedstrom oder ein Elutionsmittel zugeführt werden kann, und weiterhin über zumindest einen Auslass verfügt, über den dem gegebenenfalls zirkulierenden Flüssigkeitsstrom Lösungen der schwächer adsorbierenden Verbindung (Raffinat) oder Lösungen der stärker adsorbierenden Verbindung (Extrakt) entnommen werden können.

Beim Betrieb der SMB-Anlage werden die Einlässe und Auslässe beispielsweise über Ventile wie zum Beispiel Einzelventile, Mehrwegeventile, Ventilblöcke, Klappen oder Rotationsventile, periodisch, aber nicht notwendigerweise gleichzeitig in Richtung der Flüssigkeitsströmung weitergeschaltet, so dass scheinbar eine Gegenstrombewegung von Flüssigkeitsstrom und stationärer Phase entsteht. Dadurch kann der gegebenenfalls zirkulierende Flüssigkeitsstrom in vier Zonen eingeteilt werden, in denen die einzelnen Segmente unterschiedliche Funktion besitzen können.

In Zone I, die sich zwischen dem Einlass für das Elutionsmittel und dem Auslass für das Extrakt befindet, wird die stärker adsorbierende Verbindung von der stationären Phase desorbiert.

In Zone II, die sich zwischen dem Auslass für das Extrakt und dem Einlass für den Feedstrom befindet, wird die schwächer adsorbierende Verbindung von der stationären Phase desorbiert.

In Zone III, die sich zwischen dem Einlass für den Feedstrom und dem Auslass für das Raffinat befindet, wird die stärker adsorbierende Verbindung von der stationären Phase adsorbiert.

In Zone IV, die sich zwischen dem Auslass für das Raffinat und dem Einlass für das Elutionsmittel befindet, wird die schwächer adsorbierende Verbindung von der stationären Phase adsorbiert.

Die Zonen können beispielsweise aus einem oder mehreren Segmenten bestehen. Die Anzahl der Segmente pro Zone kann sich dabei jedoch ändern. In Sonderfällen kann es von Vorteil sein, dass eine Zone während einer Periode aus einer Flüssigkeitsverbindung jedoch nicht aus Segmenten oder Säulen besteht.

In bestimmten Fällen kann es vorteilhaft sein, die einzelnen Segmente der o.g. Vorrichtung nicht in endloser Folge (geschlossener Kreislauf), sondern in eine Serie einzelner Segmente mit einem Einlass am Beginn der Segmentserie und einem Auslass am Ende der Segmentserie hintereinander zu schalten. In diesem Fall spricht man von einem offenen Kreislauf. Dabei kann ein Teilstrom oder der Gesamtstrom des Fluids, der über den Auslass der Segmentserie gewonnen wird, direkt oder nach geeigneter Behandlung zum Einlass der Segmentserie zurückgeführt werden. Vorteilhafte Behandlungsmethoden sind z.B. Zwischenlagerung, Beprobung, Destillation, Abtrennung von Komponenten durch Membranverfahren, Mischen, Temperieren und andere.

Im Rahmen der Erfindung ist der Betrieb einer SMB-Anlage als geschlossener Kreislauf (mit zirkulierendem Flüssigkeitsstrom) bevorzugt.

Im Rahmen der Erfindung ist es vorteilhaft, eine Säulenzahl von 4 bis 24, bevorzugt 5 bis 12 und besonders bevorzugt 5 bis 8 einzusetzen.

Bevorzugt sind die Säulen als zylindrische Axialflusssäulen ausgeführt, die über eine Vorrichtung zur dynamischen Komprimierung der chiralen stationären Phase in axialer Richtung verfügen. Es können aber auch Säulen anderer Bauform eingesetzt werden.

Der Säulendurchmesser, d.h. der Durchmesser der Packung der chiralen Phase, kann beispielsweise 5 bis 1500 mm betragen, bevorzugt 50 bis 1200 mm und besonders bevorzugt 200 bis 1200 mm. Die Säulenlänge, d.h. die Länge der Packung der chiralen Phase in Flussrichtung, kann beispielsweise 15.mm bis 300 mm, bevorzugt 40 mm bis 170 mm betragen.

Es hat sich als vorteilhaft erwiesen, Säulen zu verwenden, deren Packung ein Durchmesser-Längenverhältnis von 0,25 bis 20, besonders bevorzugt 1 bis 5 aufweist.

Als chirale stationäre Phase eignen sich insbesondere solche, die Derivate von Polysacchariden, chirale Polyacrylate oder chirale Kronenether enthalten und die gegebenenfalls und vorzugsweise auf einem Trägermaterial aufgebracht sind.

Als chirale stationäre Phase eignen sich insbesondere solche, die Derivate von Polysacchariden, optisch aktive Poly(acryl)amide, optisch aktive Netzwerkpolymere oder chirale Kronenether enthalten und die gegebenenfalls und vorzugsweise auf einem Trägermaterial aufgebracht sind.

Solche gegebenenfalls auf Trägermaterialien aufgebrachten chirale stationäre Phasen sind beispielsweise bekannt aus EP-A 358 129, EP-A 1 118 623, EP-A 978 498, EP-A 625 524, EP-A 527 239 und EP-A 671 975.

Geeignete Trägermaterialien sind beispielsweise anorganische oder organische Trägermaterialien die vorzugsweise porös sind. Für den Einsatz im erfindungsgemäßen Verfahren bevorzugt sind poröse anorganische Trägermaterialien. Organische Trägermaterialien sind beispielsweise Polymere wie Polystyrole, Polyacrylsäurederivate oder deren Copolymere.

Anorganische Trägermaterialien sind beispielsweise Siliziumverbindungen wie Kieselerden, Silica-Gele und Kieselsäuren, Silikate wie Zeolithe, Aluminiumverbindungen wie Tonerden, Aluminiumoxide, Aluminate, Titanverbindungen wie Titandioxide und Titanate, Magnesiumverbindungen wie Magnesia, Gläser, Kaolin oder Apatite wie insbesondere Hydroxyapatit. Einige der genannten Trägermaterialien können in verschiedenen Modifikationen auftreten, die ebenfalls umfasst sind.

Als Trägermaterial besonders bevorzugt sind Silica-Gele.

Die Partikel des Trägermaterials weisen vorteilhafterweise einen durchschnittlichen Durchmesser (bezogen auf die Partikelzahl) von 0,1 µm bis 1 mm, bevorzugt 1 µm bis 500 µm auf.

Weiterhin weisen die Partikel des Trägermaterials vorteilhafterweise eine durchschnittliche Porengröße von 10 Å bis 50 µm auf.

Bevorzugte Polyacrylate sind solche, die die Struktureinheiten der Formel (IV) enthalten

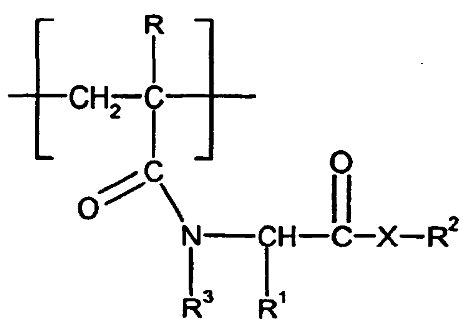

(IV),

wobei in Formel (IV)

R für Wasserstoff oder Methyl steht,

$R^1$ für eine Alkylgruppe mit 1 bis 18 C-Atomen oder eine Cycloalkylgruppe mit 3 bis 8 C-Atomen steht, die gegebenenfalls substituiert sind durch Hydroxy, Halogen, Alkoxy oder Cycloalkyl mit bis zu 8 Kohlenstoffatomen, durch eine Arylgruppe mit bis zu 14 Kohlenstoffatomen oder durch eine Heteroalkyl mit 4 bis 14 Kohlenstoffatomen, die 1 oder 2 Heteroatome aus der Gruppe Stickstoff, Sauerstoff oder Schwefel enthält, wobei die genannten

Aryl- oder Heteroarylgruppen gegebenenfalls substituiert sind durch Hydroxy, Halogen, Alkyl oder Alkoxy mit jeweils 1 bis 4 C-Atomen,

$R^3$ für Wasserstoff oder zusammen mit $R^1$ eine Tri- oder Tetramethylengruppe steht,

X für Sauerstoff oder eine $NR_4$-Gruppe steht, in der $R^4$ zusammen mit $R^2$ und dem Stickstoffatom einen 5- bis 7-gliedrigen heterocyclischen Ring bildet, der gegebenenfalls mit einer COO-Alkylgruppe (1 bis 4 C-Atome) oder durch 1 oder 2 Alkylgruppen (jeweils 1 bis 4 C-Atome) substituiert ist, und

$R^2$ für einen sperrigen stark raumerfüllenden Kohlenwasserstoffrest mit bis zu 30 Kohlenstoffatomen oder einen Heteroarylrest mit 4 bis 14 Kohlenstoffatomen steht, der 1 Heteroatom aus der Gruppe Stickstoff, Sauerstoff oder Schwefel enthält, wobei die genannten Kohlenwasserstoff- und Heteroarylreste gegebenenfalls substituiert sind durch Halogen, Hydroxy, Alkyl und/oder Alkoxy mit jeweils 1 bis 8 Kohlenstoffatomen mit der Maßgabe, dass wenn $R^2$ eine tertiäre Butylgruppe ist oder X für den Rest $NR^4$ steht, R eine Methylgruppe sein muss.

Für $R^1$ seien als gegebenenfalls substituierte Alkyl-, Cycloalkyl-, Aralkyl-, Aryl- und Heteroaryl-Reste folgende Reste vorzugsweise genannt:

als gegebenenfalls substituierte Alkylreste der Methyl-, Ethyl-, i-Propyl-, n-Propyl-, n-Butyl-, i-Butyl-, sec.-Butyl-, tert.-Butyl-, 1-Hydroxyethyl-, 2-Alkoxycarbonyl-, 3-Alkoxycarbonyl-, 3-N-Acylaminopropyl-, 4-N-Acylaminobutyl-, tert.-Butoxymethyl- und der Hydroxymethyl-Rest;

als gegebenenfalls substituierte Cycloalkylreste der Cyclohexyl- und der Tetrahydro-naphthyl-2-Rest;

als gegebenenfalls substituierte Aralkylreste der Benzyl- und 4-Hydroxybenzyl-Rest;

als gegebenenfalls substituierte Arylreste der Phenyl- und Naphthyl-Rest;

als gegebenenfalls substituierter Heteroarylrest der Indolyl-3-Rest.

Für $R^2$ seien als stark raumerfüllende Reste beispielsweise genannt:

tertiäre Alkylreste wie der tert.-Butyl-, der Neopentyl- und der Adamantyl-Rest;

in 1-Stellung durch Cycloalkylgruppen substituierte Alkylreste wie der Cyclohexylmethyl- oder Cyclohexylethyl- oder Cyclopropylmethyl-Rest;

gegebenenfalls substituierte Cycloalkylreste wie der Cyclohexyl- und die durch Methyl- oder tert.-Butylgruppen substituierten Cyclohexyl-Reste wie der 2- oder 3-Methylcyclohexyl-, der 4-tert.-Butyl- und 2,6-Di-tert.-butyl-cyclohexyl- oder der Decahydronaphthylrest.

Aralkylreste wie der 1-Phenylethyl- und der 2-Phenylpropyl-Rest;
gegebenenfalls substituierte Phenylreste wie der Phenyl- oder durch $C_1$-$C_4$-Alkylgruppen substituierte Phenylreste wie der o-Tolyl-, 2,6-Xylyl-, 4-tert.-Butyl- und 2,6-Di-tert.-butyl-phenyl-Rest;
Terpenylreste wie der Menthyl-, Neomenthyl-, Bomyl-, Fenchyl- und Pinanyl-Rest.

Besonders vorteilhaft ist die Verwendung optisch aktiver Reste für $R^2$, z.B. des d- oder 1-1-Phenylethyl- oder des d- oder 1-Methyl-, d- oder 1-Neomenthyl-, d- oder 1-Bomyl-, d- oder 1-Fenchyl- oder des d- oder 1-Pinanyl-Restes.

Die Polyacrylamide, die die Strukturelemente der Formel (VI) enthalten, sind vorzugsweise durch Polymerisation von optisch aktiven N-(Meth)Acryloylaminosäure-Derivaten der Formel (V) erhältlich

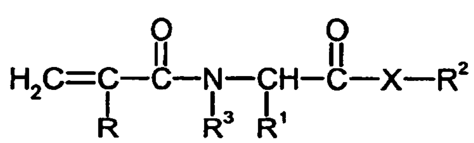

(V),

in der R, $R^1$, $R^2$ und $R^3$ die unter der Formel (IV) genannte Bedeutung besitzen. Besonders bevorzugte Polyacrylate und N-(Meth)Acryloylaminosäure-Derivaten der Formel (V) leiten sich von optisch aktiven Aminosäuren wie Alanin, Aminobuttersäure, Valin, Norvalin, Leucin, Isoleucin, Terleucin, Phenylglycin, Phenylalanin, Naphthylalanin, Cyclohexylglycin, Cyclohexylalanin, Tyrosin, Tryptophan, Threonin, Serin, Asparaginsäure, Glutaminsäure, Ornithin, Lysin oder Prolin ab.

Ganz besonders bevorzugte N-(Meth)Acryloyl-aminosäure-Derivate der Formel (I) sind: N-(Meth)Acryloylalaninmenthylester, N-(Meth)Acryloylalanin-bornylester, N-(Meth)Acryloylalanin-fenchylester, N-(Meth)Acryloylphenylalanin-methylester, N-Methacryloyl-phenylglycin-tert.-butylester, N-Methacryloyl-leucin-tert.-butylester, N-Methacryloyl-phenylalanin-tert.-butylester, N-(Meth)Acryloyl-valin-trans-4-tert. butyl-cyclohexylester, N-Methacryloyl-N'-tert.-butoxycarbonyl-lysin-tert.-butylester, N-Methacryloyl-isoleucin-tert.-butyl-ester, N-Methacryloyl-valin-tert.-butylester, N-Methacryloyl-cyclohexylalanin-tert.-butylester, N-(Meth)-Acryloyl-alanin-2-decahydronaphthylester, N-Methacryloyl-alanin-methylamid, N-Methacryloyl-phenylalanin-methylamid und N-Methacryloylphenyl-alanin-1-phenylethylamid.

Die optisch aktiven Poly(meth)acrylamide mit den Struktureinheiten der Formel (IV) liegen vorzugsweise in Form von vernetzten unlöslichen, aber quellbaren Polymerisaten oder vorzugsweise an anorganische Trägermaterialien gebundener Form vor.

Die vernetzten Polymerisate liegen weiterhin vorzugsweise in Form feinteiliger Perlen mit 5 bis 200 µm Teilchendurchmesser vor. Sie können durch Suspensionspolymerisation der optisch aktiven (Meth)Acrylamidmonomere der Formel (VI) mit 0,5 bis 50 Mol-%, vorzugsweise 1 bis 20 Mol-%, besonders bevorzugt 3 bis 15 Mol-%, (bezogen auf die Gesamtmenge (Mol) der eingesetzten Monomere) eines geeigneten Vernetzers in an sich bekannter Weise hergestellt werden.

Bevorzugte Netzwerkpolymere sind solche, die sich von optisch aktiven Diaminen, Dicarbonsäuren, Diolen oder Hydroxycarbonsäuren ableiten. Besonders bevorzugte Netzwerkpolymere sind solche, die sich von Weinsäurederivaten abgeleitet sind, die in der EP-A 671 975 offenbart sind.

Bevorzugte Kronenether sind solche der allgemeinen Formel (VI)

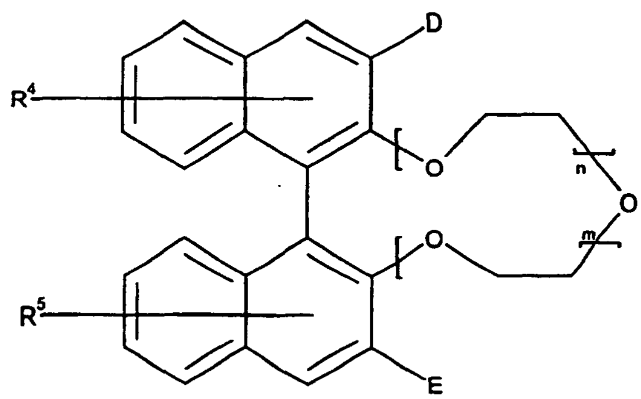

(VI)

jeweils in der R,R oder S,S-Form mit einer optischen Reinheit von mindestens 95 % ee, bevorzugt mindestens 98 % ee und besonders bevorzugt mindestens 99% ee

und in der D und E unabhängig voneinander, bevorzugt jedoch identisch für Wasserstoff, $C_1$-$C_6$-Alkyl, $C_6$-$C_{10}$-Aryl oder $C_7$-$C_{11}$-Arylalkyl stehen und

$R^4$ und $R^5$ jeweils unabhängig voneinander, bevorzugt jedoch identisch für Reste stehen, die ausgewählt sind aus der Gruppe $C_1$-$C_{30}$-Alkyl oder $C_6$-$C_{10}$-Aryl wobei die Anzahl der Reste $R^4$ und $R^5$ an der Naphthyleinheit jeweils null, eins, zwei oder drei, bevorzugt jedoch jeweils identisch null oder eins ist, wobei in diesem Fall die Substitution in 6,6'-Position bevorzugt ist und die Summe aus n+m 3 bis 10, bevorzugt 4 bis 8 und besonders bevorzugt 5 oder 6 ist.

Besonders bevorzugt sind chirale Kronenether der Formel (VI) in der D und E jeweils identisch für Phenyl stehen, n+m = 5 ist und die Anzahl der Reste $R^4$ und $R^5$ null ist.

Als stationäre chirale Phasen, die chirale Kronenether enthalten, sind solche bevorzugt, die Kronenether der Formel (VI) einschließlich der genannten Vorzugsbereiche besitzen und auf Silica-Gel aufgebracht sind. Solche chiralen Phasen sind z.B. unter den Namen Crownpak CR (+,-)® bei der Firma Daicel kommerziell verfügbar.

Bevorzugte Derivate von Polysacchariden sind solche, die sich von natürlichen oder synthetischen Glucanen, Mannanen, Galactanen, Fructanen, Xylanen oder Chitosanen ableiten.

Bevorzugt werden Derivate von solchen Polysachariden eingesetzt, die sich von Polysachariden ableiten,

die im Kettenstrang einen regelmäßigen Bindungsmodus aufweisen. Dies sind beispielsweise β-1,3-Glucane wie insbesondere Curdlan und Schizophyllan, β-1,4-Glucane wie insbesondere Cellulose, β-1,6-Glucane wie insbesondere Pustulan, α-1,2-Glucane wie insbesondere Crown-Gall-Polysaccharide, α-1,3-Glucane, α-1,4-Glucane wie insbesondere Amylose und Amylopektin oder Stärken, α-1,6-Glucane wie insbesondere Dextrane und Cyclodextrane, α-1,6-Mannane, β-1,4-Mannane, β-1,4-Galactane, β-1,2-Fructane wie insbesondere Inulin, β-2,6-Fructane wie insbesondere Levan, β-1,3-Xylane, β-1,4-Xylane, β-1,4-Chitosane, α-1,4-N-Acetylchitosane wie insbesondere Chitin. Besonders bevorzugt werden Derivate von solchen Polyssachariden eingesetzt, die sich von Cellulose, Chitin und Amylose ableiten.

Der durchschnittliche Polymerisationsgrad des Polysaccharids (Zahlenmittel) kann beispielsweise und bevorzugt bei 5 bis 500 Monosaccharideinheiten liegen ist jedoch nach oben prinzipiell nicht begrenzt.

Unter dem Begriff "Derivat von Polysacchariden" sind Polysaccharide zu verstehen, in denen die Wasserstoffatome der Hydroxygruppen oder jeweils ein Wasserstoffatom der Aminogruppen, bevorzugt jedoch die Wasserstoffatome der Hydroxygruppen zumindest teilweise durch Reste mit bis zu 30 Kohlenstoffatomen substituiert sind. Bevorzugt sind mindestens 30 %, besonders bevorzugt mindestens 50 % und ganz besonders bevorzugt mindestens 80 % der Wasserstoffatome von Hydroxygruppen oder jeweils eines Wasserstoffatoms von Aminogruppen substituiert.

Bevorzugte Reste mit bis zu 30 Kohlenstoffatomen sind solche der Formel (VIIa),

$$R^6\text{-} \qquad \text{(VIIa)},$$

in der $R^6$ für $C_4$-$C_{14}$-Aryl steht, oder solche der Formel (VIIb)

$$R^7\text{-A-CO-} \qquad \text{(VIIb)},$$

in der $R^7$ für $C_4$-$C_{14}$-Aryl steht und gleichzeitig A fehlt oder für $C_1$-$C_4$-Alkandiyl oder $C_2$-$C_4$-Alkendiyl steht oder $R^7$ für $C_1$-$C_4$-Alkyl steht und gleichzeitig A fehlt, oder solche der Formel (VIIc)

$$R^8\text{-B-NHCO-} \qquad \text{(VIIc)},$$

in der $R^8$ für $C_4$-$C_{14}$-Aryl steht und B fehlt oder für $C_1$-$C_4$-Alkandiyl steht.

$C_4$-$C_{14}$-Aryl steht dabei beispielsweise und bevorzugt für carbocyclische aromatische Reste oder heteroaromatische Reste, die keines, eins oder zwei Heteroatome pro Cyclus, im gesamten heteroaromatische Rest mindestens jedoch ein Heteroatom enthalten, die ausgewählt sind aus der Gruppe Stickstoff, Schwefel oder Sauerstoff. Weiterhin können die carbocyclischen aromatischen Reste oder heteroaromatischen Reste mit einem, zwei, drei, vier oder fünf Substituenten pro Cyclus substituiert sein, die jeweils unabhängig voneinander beispielsweise und bevorzugt ausgewählt sind aus der Gruppe $C_1$-$C_4$-Alkyl, Nitro, Cyano, O-($C_1$-$C_4$-Alkyl), Fluor, Chlor, Brom, Tri($C_1$-$C_4$-alkyl)silyl.

$C_1$-$C_4$-Alkyl steht dabei jeweils unabhängig für einen geradkettigen, verzweigten oder unverzweigten $C_1$-$C_4$-Alkyl-Rest wie beispielsweise Methyl, Ethyl, n-Propyl, iso-Propyl, n-Butyl, sec.-Butyl und tert.-Butyl.

$C_1$-$C_4$-Alkandiyl steht dabei jeweils unabhängig für einen geradkettigen, verzweigten oder unverzweigten $C_1$-$C_4$-Alkandiyl-Rest wie beispielsweise Methylen, (S)-1,1-Ethylen, (R)-1,1-Ethylen, 1,2-Propandiyl und 1,3-Propandiyl.

$C_2$-$C_4$-Alkendiyl steht dabei jeweils unabhängig für einen geradkettigen, verzweigten oder unverzweigten $C_2$-$C_4$-Alkendiyl-Rest wie beispielsweise Ethenyl, 1,2-Propenyl und 1,3-Propenyl.

In Formel (VIIa) steht $R^6$ bevorzugt für Phenyl, das mit keinem einem oder zwei Resten substituiert ist, die jeweils unabhängig ausgewählt sind aus der Gruppe Methyl, Nitro, Chlor, Brom, Tri($C_1$-$C_4$-alkyl)silyl

In Formel (VIIb) stehen $R^7$ und A zusammen bevorzugt für Methyl, 2-Phenylethenyl, Phenyl oder p-Tolyl.

In Formel (VIIc) stehen $R^8$ und B zusammen bevorzugt für (S) oder (R)-Phenylethyl, Phenyl, 3,5-Dimethylphenyl, p-Tolyl und p-Chlorphenyl, wobei 3,5-Dimethylphenyl noch weiter bevorzugt ist.

Als stationäre chirale Phasen sind im Rahmen der Erfindung solche bevorzugt, die chirale Kronenether der Formel (VI) einschließlich der genannten Vorzugsbereiche besitzen und auf Silica-Gel aufgebracht sind. Solche chiralen Phasen sind z.B. unter den Namen Crownpak CR (+,-)® von der Firma Daicel kommerziell verfügbar.

Weiterhin sind als stationäre chirale Phasen im Rahmen der Erfindung solche bevorzugt, die Derivate von Polysacchariden einschließlich der genannten Vorzugsbereiche besitzen und auf Silica-Gel aufgebracht sind. Solche chiralen Phasen sind z.B. unter den Namen Chiralpak ® (AD,AS)™ oder Chiralcel ® (OD,OJ,OA,OB,OC, OF, OG, OK) ™ von der Firma Daicel kommerziell verfügbar.

Besonders bevorzugt werden als stationäre chirale Phasen im Rahmen der Erfindung solche verwendet, die Amylose-tris(3,5-dimethylphenylcarbamat), Amylose-tris-[(S)-α-methyl-benzylcarbamat], Cellulose-tris(3,5-dimethylphenylcarbamat), Cellulose-tris(4-methylbenzoat) oder Cellulose-tris(4-chlorphenylcarbamat) enthalten und auf Silica-Gel aufgebracht sind (z.B. erhältlich unter dem Namen Chiralpak® (AD,AS)™ oder Chiralcel (OD,OJ, OF)™ von der Firma Daicel).

Ganz besonders bevorzugt sind als stationäre chirale Phasen im Rahmen der Erfindung solche, die Amylose-tris(3,5-dimethylphenylcarbamat) oder Cellulose-tris(3,5-dimethylphenylcarbamat) enthalten und auf Silica-Gel aufgebracht sind (z.B. erhältlich unter dem Namen Chiralpak® (AD)™ oder Chiralcel® (OD)™ bei der Firma Daicel), wobei als stationäre chirale Phasen solche noch weiter bevorzugt sind, die Amylose-tris(3,5-dimethylphenylcarbamat) enthalten und auf Silica-Gel aufgebracht [Chiralpak® (AD)™].

Das für die Enantiomerentrennung eingesetzte Ausgangsgemisch, das die Enantiomeren von cis-8-Benzyl-7,9-dioxo-2,8-diazabicyclo[4.3.0]nonan enthält, wird in einem Lösungsmittel gelöst als sogenannter Feedstrom dem zirkulierenden Flüssigkeitsstrom zugeführt.

Der Anteil des Ausgangsgemischs am Feedstrom kann beispielsweise 1 bis 35 Masse-%, bevorzugt 5 bis 30 Masse-% und besonders bevorzugt 15 bis 30 Masse-% betragen.

Als Lösungsmittel sind organische Lösungsmittel geeignet. Das sind beispielsweise und bevorzugt aliphatische Kohlenwasserstoffe mit 6 bis 12 Kohlenstoffatomen wie vorzugsweise Methyl-cyclohexan, Cyclohexan, n-Hexan und n-Heptan, Ether wie vorzugsweise Tetrahydrofuran, aliphatische Alkohole mit 1 bis 6 Kohlenstoffatomen wie vorzugsweise Methanol, Ethanol und Iso-Propanol, Nitrile wie vorzugsweise Acetonitril, Benzonitril und Benzylnitriloder Mischungen solcher Lösungsmittel.

Besonders bevorzugt sind n-Hexan, n-Heptan, Iso-Propanol und Acetonitril oder Mischungen davon, wobei Acetonitril noch weiter bevorzugt ist.

Dem gegebenenfalls zirkulierenden Flüssigkeitsstrom wird weiterhin ein Elutionsmittel zugeführt. Das Elutionsmittel ist vorteilhafterweise ein organisches Lösungsmittel, wobei die oben genannten Angaben einschließlich der Vorzugsbereiche in gleicher Weise gelten. Besonders bevorzugt werden für den Feedstrom und das Elutionsmittel die gleichen Lösungsmittel eingesetzt.

Werden Mischungen von Lösungsmitteln eingesetzt, ist es weiterhin möglich, während der Zuführung des Elutionsmittels die Zusammensetzung zu ändern, was beispielsweise intervallweise oder in Form eines Gradienten kontinuierlich erfolgen kann.

Im Rahmen der Erfindung ist es jedoch bevorzugt, bei konstanter Zusammensetzung des Lösungsmittels zu arbeiten. Noch weiter bevorzugt ist die Verwendung nur eines Lösungsmittels.

Vorteilhafterweise werden organische Lösungsmittel verwendet, die einen Wassergehalt von 3 Masse-% oder weniger, bevorzugt 0,3 Masse-% oder weniger und besonders bevorzugt 0,03 Masse-% oder weniger aufweisen.

Der Druck bei der Zuführung des Feedstroms und des Elutionsmittels kann beispielsweise 0,5 bar bis 100 bar betragen, bevorzugt sind 1 bar bis 60 bar.

Die Temperatur bei der Enantiomerenanreicherung kann beispielsweise 0 bis 80°C betragen, bevorzugt 10 bis 40°C, besonders bevorzugt 18 bis 32°C und ganz besonders bevorzugt 20 bis 28°C.

Der SMB-Anlage kann dann das Raffinat und der Extrakt entnommen werden, wobei diese Fraktionen jeweils ein angereichertes Enantiomer des cis-8-Benzyl-7,9-dioxo-2,8-diazabicyclo[4.3.0]nonans enthalten, wobei die angereicherten Enantiomere durch Entfernen des Lösungsmittels beispielsweise durch Verdampfen gewonnen werden können.

Auf erfindungsgemäße Weise können die Enantiomere des cis-8-Benzyl-7,9-dioxo-2,8-diazabicyclo[4.3.0]nonans, insbesondere das (1S,6R)-8-Benzyl-7,9-dioxo-2,8-diazabicyclo[4.3.0]nonan beispielsweise mit optischen Reinheiten von 70 % ee oder mehr, bevorzugt 85 % ee oder mehr, besonders bevorzugt 90 % ee oder mehr und ganz besonders bevorzugt 95 % ee oder mehr erhalten werden.

Auf erfindungsgemäße Weise können das Enantiomer des Raffinats, vorzugsweise das (1S,6R)-8-Benzyl-7,9-dioxo-2,8-diazabicyclo[4.3.0]nonan, beispielsweise mit absoluten Reinheiten von 90 % oder mehr, bevorzugt 95 % oder mehr und besonders bevorzugt 98 % oder mehr erhalten werden.

Weiterhin kann auf erfindungsgemäße Weise das Enantiomer des Extrakts, vorzugsweise das (1R,6S)-8-Benzyl-7,9-dioxo-2,8-diazabicyclo[4.3.0]nonan beispielsweise mit absoluten Reinheiten von 85 % oder mehr, bevorzugt 90 % oder mehr und besonders bevorzugt 95 % oder mehr erhalten werden.

Die Ausbeuten bezogen auf die maximal gewinnbare Menge der Enantiomere des cis-8-Benzyl-7,9-dioxo-2,8-diazabicyclo[4.3.0]nonans, insbesondere das (1S,6R)-8-Benzyl-7,9-dioxo-2,8-diazabicyclo[4.3.0]nonan können 60 % oder mehr, bevorzugt 80 % oder mehr und besonders bevorzugt 95 % oder mehr betragen.

Ein besondere Eigenschaft des erfindungsgemäßen Verfahrens ist es, dass die Enantiomere des cis-8-Benzyl-7,9-dioxo-2,8-diazabicyclo[4.3.0]nonans, insbesondere das (1S,6R)-8-Benzyl-7,9-dioxo-2,8-diazabicyclo[4.3.0]nonan in optischen Reinheiten von 95%ee oder mehr bei Ausbeuten bezogen auf die maximal gewinnbare Menge des Enantiomers von über 95% gewonnen werden können.

Weiterhin können die Enantiomere des cis-8-Benzyl-7,9-dioxo-2,8-diazabicyclo[4.3.0]nonans, insbesondere das (1S,6R)-8-Benzyl-7,9-dioxo-2,8-diazabicyclo[4.3.0]nonan bei optischen Reinheiten von 90 %ee oder mehr mit einer Produktivität von über 0,2 kg, bevorzugt über 0,8 kg und besonders bevorzugt über 3,0 kg pro kg chirale stationäre Phase und Tag [kg/($kg_{CSP}$● d)] erhalten werden.

Ist für einen Folgeschritt nur ein Enantiomeres von Interesse, so ist es vorteilhaft, das angereicherte unerwünschte Enantiomere zu racemisieren und erneut der kontinuierlichen Gegenstromchromatographie zuzuführen.

In einer bevorzugten Ausführungsform wird das (1S,6R)-8-Benzyl-7,9-dioxo-2,8-diazabicyclo[4.3.0]nonan aus dem Raffinat gewonnen und das Enantiomere (1R,6S)-8-Benzyl-7,9-dioxo-2,8-diazabicyclo[4.3.0]nonan racemisiert und erneut der kontinuierlichen Gegenstromchromatographie zugeführt.

Die Racemisierung ist prinzipiell aus der EP-A 1067 129 bekannt und erfolgt durch Zusatz von Base.

Dabei kann zur Racemisierung beispielsweise reines (1R,6S)-8-Benzyl-7,9-dioxo-2,8-diazabicyclo[4.3.0]nonan oder Gemische eingesetzt werden, die beispielsweise über 70 Gew.-%, vorzugsweise über 85 Gew.-% (1R,6S)-8-Benzyl-7,9-dioxo-2,8-diazabicyclo[4.3.0]nonan enthalten. Ergänzend zu 100 Gew.-% können diese Gemische beispielsweise (1S,6R)-8-Benzyl-7,9-dioxo-2,8-diazabicyclo[4.3.0]nonan enthalten.

Weiterhin ist es möglich und bevorzugt, das zu racemisierende Enantiomer, bevorzugt (1R,6S)-8-Benzyl-7,9-dioxo-2,8-diazabicyclo[4.3.0]nonan, direkt in Form der Raffinat- oder Extrakt-Lösung aus der Enantiomerenanreicherung einzusetzen. Üblicherweise fällt (1R,6S)-8-Benzyl-7,9-dioxo-2,8-diazabicyclo[4.3.0]nonan in Form der Extrakt-Lösung an.

Gegebenenfalls kann die Raffinat- oder Extrakt-Lösung durch Verdampfen von Lösungsmittel aufkonzentriert werden.

Als Basen kommen für die Racemisierung beispielsweise Alkoholate der Formel (VIII) in Frage,

$$MOR^9 \quad \text{(VIII)},$$

in der

M für Lithium, Natrium oder Kalium, vorzugsweise für Natrium oder Kalium und

$R^9$ für geradkettiges oder verzweigtes $C_1$-$C_6$-Alkyl, vorzugsweise für Methyl oder tert.-Butyl stehen.

Bevorzugte Einzelverbindungen der Formel (VIII) sind Natriummethylat, Natrium-tert.-butylat und Kalium-tert.-butylat. Besonders bevorzugt ist Kalium-tert.-butylat.

Bevorzugt wird die Base in einer Menge von 1 bis 20 Mol bezogen auf die Menge des zu racemisierenden cis-8-Benzyl-7,9-dioxo-2,8-diazabicyclo[4.3.0]nonans eingesetzt.

Die Alkoholate können in fester Form oder gelöst in einem Lösungsmittel zugeführt werden. Als Lösungsmittel kommen z.B. Alkohole und aprotische Lösungsmittel in Frage, beispielsweise der Alkohol, der dem jeweils eingesetzten Alkoholat entspricht, und geradkettige, verzweigte und cyclische Ether sowie aromatische Kohlenwasserstoffe. Einzelbeispiele für aprotische Lösungsmittel sind: Methyl-tert.-butylether, Tetrahydrofuran, Dioxan, Toluol und Xylol. Bevorzugte Alkoholatlösungen sind: Kalium-tert.-butylat in tert.-Butanol und in Tetrahydrofuran und Natriummethylat in Methanol.

In einer bevorzugten Ausführungsform wird das Alkoholat in dem Lösungsmittel zugeführt, das als Elutionsmittel in der chromatographischen Trennung dient.

Die Racemisierung kann z.B. bei Temperaturen zwischen -10 und 40°C durchgeführt werden.

Die erfindungsgemäße Racemisierung ist im Allgemeinen nach spätestens 5 Stunden beendet. Unter geeigneten Reaktionsbedingungen (z.B. entsprechender Wahl der Base, des Lösungsmittels und der Temperatur) kann die erforderliche Reaktionszeit wesentlich kürzer sein und beispielsweise 15 Minuten oder noch weniger betragen.

Die Aufarbeitung des nach der Racemisierung vorliegenden Reaktionsgemisches kann so erfolgen, dass man zunächst die eingesetzte Base neutralisiert, z.B. durch Zusatz einer organischen Säure, z.B. einer $C_1$-$C_6$-Carbonsäure, einer Mineralsäure, z.B. Schwefelsäure oder Phosphorsäure, von Kohlensäure oder eines sauren Ionenaustauschers. Die Menge der Säure bzw. des sauren Ionenaustauschers kann z.B. 0,9 bis 1,1 Äquivalente pro Äquivalent eingesetzte Base betragen. Vorzugsweise liegt diese Menge bei 0,97 bis 1,03 Äquivalenten pro Äquivalent eingesetzte Base, insbesondere setzt man die Säure bzw. den sauren Ionenaustauscher in äquivalenter Menge ein, bezogen auf die eingesetzte Base. Danach kann man das Lösungsmittel entfernen, z.B. durch Destillation, gegebenenfalls unter vermindertem Druck. Es hinterbleibt dann ein Racemisierungsgemisch, das die Enantiomeren cis-8-Benzyl-7,9-dioxo-2,8-diazabicyclo[4.3.0]nonane in einem molaren Verhältnis von 1:1 bis 1,5:1, bevorzugt 1:1 bis 1,1:1 enthält, wobei natürlich nur dasjenige Enantiomer im Überschuss vorliegen kann, das in angereicherter Form zur Racemisierung

eingesetzt wurde.

Werden vor, während oder nach der Durchführung Lösungsmittel entfernt, die als Elutionsmittel eingesetzt wurden, können diese für das erfindungsgemäße Verfahren erneut eingesetzt werden.

Das Racemisierungsgemisch kann dann entweder gelagert oder der Enantiomerenanreicherung wieder zugeführt werden. Dieser Prozessablauf Enantiomerenanreicherung, Racemisierung, Enantiomerenanreicherung kann gegebenenfalls unter ständiger Neuzuführung von Ausgangsgemisch beliebig oft wiederholt werden, so dass auf erfindungsgemäße Weise letztendlich beispielsweise das bei der Kernhydrierung von Pyridin-2,3-dicarbonsäure-N-benzylimid anfallende, racemische Gemisch der cis-8-Benzyl-7,9-dioxo-2,8-diazabicyclo[4.3.0]nonane vollständig in ein angereichertes Enantiomer, bevorzugt (1S,6R)8-Benzyl-7,9-dioxo-2,8-diazabicyclo[4.3.0]nonan mit hoher absoluter und optischer Reinheit überführt werden kann.

Die auf erfindungsgemäße Weise erhältlichen, angereicherten Enantiomere (1S,6R)-8-Benzyl-7,9-dioxo-2,8-diazabicyclo[4.3.0]nonan und (1R,6S)-8-Benzyl-7,9-dioxo-2,8-diazabicyclo[4.3.0]nonan eignen sich insbesondere in einem Verfahren zur Herstellung von antibakteriellen Mitteln und Futterzusatzmitteln.

Weiterhin eignet sich das auf erfindungsgemäße Weise erhältliche, angereicherte Enantiomere (1S,6R)-8-Benzyl-7,9-dioxo-2,8-diazabicyclo[4.3.0]nonan zur Herstellung von (S,S)-2,8-diazabicyclo[4.3.0]nonan und Moxifloxacin (INN, 1-Cyclopropyl-7-([S,S]-2,8-diazabicyclo[4.3.0]non-8-yl)-6-fluor-1,4-dihydro-8-methoxy-4-oxo-3-chinoloncarbonsäure, das angereicherte Enantiomere (1R,6S)-8-Benzyl-7,9-dioxo-2,8-diazabicyclo[4.3.0]nonan zur Herstellung von (R,R)-2,8-diazabicyclo-[4.3.0]nonan.

Das erfindungsgemäße Verfahren zeichnet sich dadurch aus, dass in einem kontinuierlichen Prozess (1S, 6R)-8-Benzyl-7,9-dioxo-2,8-diazabicyclo[4.3.0]nonan und (1R,6S)-8-Benzyl-7,9-dioxo-2,8-diazabicyclo[4.3.0]nonan in hoher Reinheit, optischer Reinheit und hoher Ausbeute mit unerwarteter Produktivität gewonnen werden kann. Durch ein nachgeschaltete Racemisierung und Rückführung in den Trennprozess kann weiterhin in besonders vorteilhafter Weise ein einziges Zielenantiomer, insbesondere (1S,6R)-8-Benzyl-7,9-dioxo-2,8-diazabicyclo[4.3.0]nonan in höchstzeffizienter Weise gewonnen werden.

## Beispiele

### Beispiel 1

Es wurde eine kontinuierlich arbeitende Gegenstrom-Chromatographieanlage der Firma NovaSep, Frankreich, eingesetzt (Typ: LICOSEP 8-200). Charakteristischer Bestandteil der Anlage sind 8 Axialflusssäulen (Durchmesser jeweils 200 mm) mit einer Vorrichtung zur dynamischen Axialkomprimierung der stationären Phase.

Alle äußeren Zu- und Abläufe werden über entsprechende Anschlüsse jeweils an bestimmten Stellen zu- bzw. abgeführt, die mit dem umlaufenden Konzentrationsprofil korrespondierenden. Es wurden folgende Mengenströme eingestellt:

| | |
|---|---|
| Zone I | 500 l/h |
| Zone II | 260,0 l/h |
| Zone III | 274,5 l/h |
| Zone IV | 200,0 l/h |
| Elutionsmittel | 300 l/h (Acetonitril, Wassergehalt: < 300 ppm) |
| Feedstrom | 14,5 l/h |
| mit 199,4 mg rac-DOPP/ml (Lösungsmittel: Acetonitril, Wassergehalt < 300 ppm) | |
| Raffinat | 74,5 l/h (1S,6R)-8-Benzyl-7,9-dioxo-2,8-diazabicyclo[4.3.0]nonan in Acetonitril |
| Extrakt | 240,0 l/h (1R,6S)-8-Benzyl-7,9-dioxo-2,8-diazabicyclo[4.3.0]nonan in Acetonitril |
| Taktzeit | 0,72 min |
| Temperatur | 25 °C |
| Chirale stationäre Phase | Chiralpak® AD™, 20µm |

In der nachfolgenden Tabelle 1 sind die Zusammensetzungen des Feedstroms und des Extrakts angegeben. Die Ausbeute ist quantitativ.

Tabelle 1

| Extrakt | | Raffinat | | Produktivität |
|---|---|---|---|---|
| %ee | %* | %ee | %* | $kg_{rac}/kg_{CSP}/d$ |
| 95,6 | 96,4 | 98,4 | 98,8 | 5,42 |

* Produktstrom ohne Lösungs- bzw. Elutionsmittel

**Beispiel 2**

Es wurde eine kontinuierlich arbeitende Gegenstrom-Chromatographieanlage der Firma NovaSep, Frankreich, eingesetzt (Typ: LICOSEP 8-200). Charakteristischer Bestandteil der Anlage sind 7 Axialflusssäulen (Durchmesser jeweils 200 mm) mit einer Vorrichtung zur dynamischen Axialkomprimierung der stationären Phase, die der Konfiguration 2:2:2:1 (Zone1: Zone2:Zone3:Zone4) zum Einsatz kamen.

Alle äußeren Zu- und Abläufe werden über entsprechende Anschlüsse jeweils an bestimmten Stellen zu- bzw. abgeführt, die mit dem umlaufenden Konzentrationsprofil korrespondierenden. Es wurden folgende Mengenströme eingestellt:

| | |
|---|---|
| Zone I | 500 l/h |
| Zone II | 286,5 l/h |
| Zone III | 297,0 l/h |
| Zone IV | 200,0 l/h |
| Elutionsmittel | 300 l/h (Acetonitril, Wassergehalt; < 300 ppm) |
| Feedstrom | 10,5 l/h |
| mit 202,7 mg rac-DOPP/ml (Lösungsmittel: Acetonitril, Wassergehalt < 300 ppm) | |
| Raffinat | 97 l/h (1S,6R)-8-Benzyl-7,9-dioxo-2,8-diazabicyclo[4.3.0]nonan in Acetonitril |
| Extrakt | 213,5 l/h (1R,6S)-8-Benzyl-7,9-dioxo-2,8-diazabicyclo[4.3.0]nonan in Acetonitril |
| Taktzeit | 0,69 min |
| Temperatur | 27°C |
| Chirale stationäre Phase | Chiralpak® AD™ , 20µm |

In der nachfolgenden Tabelle 2 sind die Zusammensetzungen des Feedstroms und des Extrakts angegeben. Die Ausbeute ist quantitativ.

Tabelle 2

| Extrakt | | Raffinat | | Produktivität |
|---|---|---|---|---|
| %ee | %* | %ee | %* | $kg_{rac}/kg_{CSP}/d$ |
| >99,9 | 96,3 | >99,9 | 98,9 | 4,56 |

* Produktstrom ohne Lösungs- bzw. Elutionsmittel

## Patentansprüche

1. Verfahren zur Enantiomerenanreicherung von cis-8-Benzyl-7,9-dioxo-2,8-diazabicyclo[4.3.0]nonan, **dadurch gekennzeichnet, dass** die Enantiomerenanreicherung durch kontinuierliche Gegenstromchromatographie erfolgt.

2. Verfahren gemäß Anspruch 1, **dadurch gekennzeichnet, dass** die Enantiomerenanreicherung mit Hilfe einer SMB-Anlage durchgeführt wird.

3. Verfahren gemäß Anspruch 2, **dadurch gekennzeichnet, dass** in der SMB-Anlage durch zwei oder mehr miteinander verbundene Segmente ein sich in einer Richtung bewegender und gegebenenfalls zirkulierender Flüssigkeitsstrom erzeugt wird, wobei jedes Segment über mindestens eine mit einer chiralen stationären Phase befüllte Säule verfügt und in Strömungsrichtung zumindest mit einem Flüssigkeitseinlass und einem Flüssigkeitsauslass versehen ist und wobei jedes Segment über zumindest einen Einlass verfügt, über den dem gegebenenfalls zirkulierenden Flüssigkeitsstrom ein Feedstrom oder ein Elutionsmittel zugeführt werden kann, weiterhin über zumindest einen Auslass verfügt, über den dem gegebenenfalls zirkulierenden Flüssigkeitsstrom Lösungen der schwächer adsorbierenden Verbindung (Raffinat) oder Lösungen der stärker adsorbierenden Verbindung (Extrakt) entnommen werden können.

4. Verfahren gemäß einem oder mehreren der Ansprüche 2 bis 3, **dadurch gekennzeichnet, dass** die SMB-Anlage eine Säulenzahl von 4 bis 24 aufweist.

5. Verfahren gemäß einem oder mehreren der Ansprüche 2 bis 4, **dadurch gekennzeichnet, dass** die SMB-Anlage Säulen enthält, die als zylindrische Axialflusssäulen ausgeführt sind und über eine Vorrichtung zur dynamischen Komprimierung verfügen.

6. Verfahren gemäß einem oder mehreren der Ansprüche 2 bis 5, **dadurch gekennzeichnet, dass** die SMB-Anlage Säulen enthält, die einen Säulendurchmesser von 5 bis 1500 mm besitzen.

7. Verfahren gemäß einem oder mehreren der Ansprüche 2 bis 6, **dadurch gekennzeichnet, dass** die SMB-Anlage Säulen enthält, die eine Säulenlänge von 15 mm bis 300 mm besitzen.

8. Verfahren gemäß einem oder mehreren der Ansprüche 3 bis 7, **dadurch gekennzeichnet, dass** in der SMB-Anlage ein geschlossener Kreislauf mit zirkulierendem Flüssigkeitsstrom erzeugt wird.

9. Verfahren gemäß einem oder mehreren der Ansprüche 1 bis 8, **dadurch gekennzeichnet, dass** zur Enantiomerenanreicherung ein Ausgangsgemisch eingesetzt wird, das die Enantiomeren von cis-8-Benzyl-7,9-dioxo-2,8-diazabicyclo[4.3.0]nonan in einem molaren Verhältnis von 0,25:1 bis 4:1 enthält.

10. Verfahren gemäß einem oder mehreren der Ansprüche 1 bis 9, **dadurch gekennzeichnet, dass** das cis-8-Benzyl-7,9-dioxo-2,8-diazabicyclo[4.3.0]-nonan durch Kernhydrierung von Pyridin-2,3-dicarbonsäure-N-benzylimid erhalten wird.

11. Verfahren gemäß Anspruch 10, **dadurch gekennzeichnet, dass** die Enantiomerenanreicherung in Gegenwart von trans-8-Benzyl-7,9-dioxo-2,8-diazabicyclo-[4.3.0]nonan bzw. dessen Enantiomeren und/oder anderen aus der Kernhydrierung von Pyridin-2,3-dicarbonsäure-N-benzylimid stammenden organischen Nebenprodukten durchgeführt wird.

12. Verfahren gemäß einem oder mehreren der Ansprüche 1 bis 11, **dadurch gekennzeichnet, dass** zur Enantiomerenanreicherung Säulen eingesetzt werden, die als chirale stationäre Phase Derivate von Polysacchariden, chirale Polyacrylate, oder chirale Kronenether enthalten, die gegebenenfalls auf einem Trägermaterial aufgebracht sind.

13. Verfahren gemäß Anspruch 1 bis 12, **dadurch gekennzeichnet, dass** als chirale stationäre Phasen solche verwendet werden, die Amylose-tris(3,5-dimethyl-phenylcarbamat), Amylose-tris-[(S)-α-methyl-benzylcarbamat], Cellulose-tris(3,5-dimethylphenylcarbamat), Cellulose-tris(4-methylbenzoat) oder Cellulose-tris(4-chlorphenylcarbamat) enthalten und auf Silica-Gel aufgebracht sind.

14. Verfahren gemäß einem oder mehreren der Ansprüche 12 bis 13, **dadurch gekennzeichnet, dass** als chirale stationäre Phase solche verwendet werden, die Amylose-tris(3,5-dimethylphenylcarbamat) oder Cellulose-tris (3,5-dimethyl-phenylcarbamat) enthalten und auf Silica-Gel aufgebracht sind.

15. Verfahren nach einem oder mehreren der Ansprüche 1 bis 14, **dadurch gekennzeichnet, dass** als Lösungsmittel aliphatische Kohlenwasserstoffe mit 6 bis 12 Kohlenstoffatomen, Ether, aliphatische Alkohole mit 1 bis 6 Kohlenstoffatomen, Nitrile oder Mischungen solcher Lösungsmittel eingesetzt werden.

16. Verfahren nach einem oder mehreren der Ansprüche 1 bis 15, **dadurch gekennzeichnet, dass** als Lösungsmittel Acetonitril eingesetzt wird.

17. Verfahren nach einem oder mehreren der Ansprüche 1 bis 16, **dadurch gekennzeichnet, dass** als Lösungsmittel solche verwendet werden, die einen Wassergehalt von 3 Masse-% oder weniger aufweisen.

18. Verfahren nach einem oder mehreren der Ansprüche 2 bis 17, **dadurch gekennzeichnet, dass** der Druck bei der Zuführung des Feedstroms und des Elutionsmittels 0,5 bar bis 100 bar beträgt.

**19.** Verfahren nach einem oder mehreren der Ansprüche 1 bis 18, **dadurch gekennzeichnet, dass** die Temperatur bei der Enantiomerenanreicherung 0 bis 80°C beträgt.

**20.** Verfahren nach einem oder mehreren der Ansprüche 1 bis 19, **dadurch gekennzeichnet, dass** die Enantiomere des cis-8-Benzyl-7,9-dioxo-2,8-diazabicyclo[4.3.0]nonans mit optischen Reinheiten von 70 % ee oder mehr erhalten werden.

**21.** Verfahren nach einem oder mehreren der Ansprüche 1 bis 20, **dadurch gekennzeichnet, dass** die Enantiomere des cis-8-Benzyl-7,9-dioxo-2,8-diazabicyclo-[4.3.0]nonans bei optischen Reinheiten von 90% ee oder mehr mit einer Produktivität von über 0,2 kg pro kg chirale stationäre Phase und Tag erhalten werden.

**22.** Verfahren nach einem oder mehreren der Ansprüche 1 bis 21, **dadurch gekennzeichnet, dass** das angereicherte, unerwünschte Enantiomere racemisiert wird.

**23.** Verfahren nach Anspruch 22, **dadurch gekennzeichnet, dass** das angereicherte unerwünschte Enantiomere racemisiert und erneut der kontinuierlichen Gegenstromchromatographie zugeführt wird.

**24.** Verfahren nach einem oder mehreren der Ansprüche 22 bis 23, **dadurch gekennzeichnet, dass** das (1S,6R)-8-Benzyl-7,9-dioxo-2,8-diazabicyclo[4.3.0]nonan gewonnen und das Enantiomere (1R,6S)-8-Benzyl-7,9-dioxo-2,8-diazabicyclo[4.3.0]nonan racemisiert und erneut der kontinuierlichen Gegenstromchromatographie zugeführt wird.

## Claims

**1.** Process for the enantiomeric enrichment of cis-8-benzyl-7,9-dioxo-2,8-diazabicyclo[4.3.0]nonane, **characterized in that** the enantiomeric enrichment is carried out by continuous countercurrent chromatography.

**2.** Process according to Claim 1, **characterized in that** the enantiomeric enrichment is carried out with the aid of an SMB unit.

**3.** Process according to Claim 2, **characterized in that** a stream of liquid moving in one direction and optionally circulating is produced in the SMB unit by means of two or more segments connected to one another, each segment having at least one column filled with a chiral stationary phase and being provided in the flow direction at least with a liquid inlet and a liquid outlet and each segment having at least one inlet, via which a feedstream or an eluting agent can be fed to the optionally circulating stream of liquid, furthermore having at least one outlet, via which solutions of the more weakly adsorbing compound (raffinate) or solutions of the more strongly adsorbing compound (extract) can be removed from the optionally circulating stream of liquid.

**4.** Process according to one or more of Claims 2 and 3, **characterized in that** the SMB unit has a column number of 4 to 24.

**5.** Process according to one or more of Claims 2 to 4, **characterized in that** the SMB unit contains columns which are designed as cylindrical axial flow columns and have a device for dynamic compression.

**6.** Process according to one or more of Claims 2 to 5, **characterized in that** the SMB unit contains columns which have a column diameter of 5 to 1500 mm.

**7.** Process according to one or more of Claims 2 to 6, **characterized in that** the SMB unit contains columns which have a column length of 15 mm to 300 mm.

**8.** Process according to one or more of Claims 3 to 7, **characterized in that** a closed circulation with a circulating stream of liquid is produced in the SMB unit.

**9.** Process according to one or more of Claims 1 to 8, **characterized in that**, for the enantiomeric enrichment, a starting mixture is employed which contains the enantiomers of cis-8-benzyl-7,9-dioxo-2,8-diazabicyclo[4.3.0]nonane in a molar ratio of 0.25:1 to 4:1.

**10.** Process according to one or more of Claims 1 to 9, **characterized in that** the cis-8-benzyl-7,9-dioxo-2,8-diazabicyclo[4.3.0]nonane is obtained by nuclear hydrogenation of pyridine-2,3-dicarboxylic acid-N-benzylimide.

**11.** Process according to Claim 10, **characterized in that** the enantiomeric enrichment is carried out in the presence of trans-8-benzyl-7,9-dioxo-2,8-diazabicyclo[4.3.0]nonane or its enantiomers and/or other organic by-products originating from the nuclear hydrogenation of pyridine-2,3-dicarboxylic acid N-benzylimide.

**12.** Process according to one or more of Claims 1 to 11, **characterized in that**, for the enantiomeric enrichment, columns are employed which, as a chiral stationary phase, contain polysaccharides, chiral polyacrylates, or chiral crown ethers, which are optionally applied to a support material.

**13.** Process according to Claims 1 to 12, **characterized in that**, as chiral stationary phases, those are used which contain amylose tris(3,5-dimethyl-phenylcarbamate), amylose tris[(S)-$\alpha$-methylbenzylcarbamate], cellulose tris (3,5-dimethylphenylcarbamate), cellulose tris(4-methylbenzoate) or cellulose tris(4-chlorphenylcarbamate) and are applied to silica gel.

**14.** Process according to one or more of Claims 12 and 13, **characterized in that**, as a chiral stationary phase, those are used which contain amylose tris(3,5-dimethylphenylcarbamate) or cellulose tris(3,5-dimethylphenylcarbamate) and are applied to silica gel.

**15.** Process according to one or more of Claims 1 to 14, **characterized in that** the solvents employed are aliphatic hydrocarbons having 6 to 12 carbon atoms, ethers, aliphatic alcohols having 1 to 6 carbon atoms, nitriles or mixtures of such solvents.

**16.** Process according to one or more of Claims 1 to 15, **characterized in that** the solvent employed is acetonitrile.

**17.** Process according to one or more of Claims 1 to 16, **characterized in that** the solvents used are those which have a water content of 3 mass% or less.

**18.** Process according to one or more of Claims 2 to 17, **characterized in that** the pressure during the feed of the feedstream and of the eluting agent is 0.5 bar to 100 bar.

**19.** Process according to one or more of Claims 1 to 18, **characterized in that** the temperature during the enantiomeric enrichment is 0 to 80°C.

**20.** Process according to one or more of Claims 1 to 19, **characterized in that** the enantiomers of cis-8-benzyl-7,9-dioxo-2,8-diazabicyclo[4.3.0]nonane having optical purities of 70% ee or more are obtained.

**21.** Process according to one or more of Claims 1 to 20, **characterized in that** the enantiomers of cis-8-benzyl-7,9-dioxo-2,8-diazabicyclo[4.3.0]nonanes are obtained with optical purities of 90% ee or more having a productivity of over 0.2 kg per kg of chiral stationary phase per day.

**22.** Process according to one or more of Claims 1 to 21, **characterized in that** the enriched, undesired enantiomer is racemized.

**23.** Process according to Claim 22, **characterized in that** the enriched undesired enantiomer is racemized and fed again to the continuous countercurrent chromatography.

**24.** Process according to one or more of Claims 22 to 23, **characterized in that** (1S,6R)-8-benzyl-7,9-dioxo-2,8-diazabicyclo[4.3.0]nonane is obtained and the enantiomer (1R,6S)-8-benzyl-7,9-dioxo-2,8-diazabicyclo[4.3.0]nonane is racemized and fed again to the continuous countercurrent chromatography.

## Revendications

**1.** Procédé pour enrichir le cis-8-benzyl-7,9-dioxo-2,8-diazabicyclo[4.3.0]nonane en un énantiomère, **caractérisé en ce que** cet enrichissement est réalisé par chromatographie continue à contre-courant.

**2.** Procédé selon revendication 1, **caractérisé en ce que** l'enrichissement en un énantiomère est réalisé à l'aide d'une installation SMB.

**3.** Procédé selon revendication 2, **caractérisé en ce que**, dans l'installation SMB, au travers de deux segments ou plus, reliés entre eux, on produit un courant de liquide se déplaçant dans une direction et le cas échéant soumis à circulation, chaque segment comportant au moins une colonne garnie d'une phase stationnaire chirale et, dans le sens de l'écoulement, au moins une entrée de liquide et une sortie de liquide, chaque segment comportant au moins une entrée par laquelle on peut amener dans le courant de liquide éventuellement, en circulation, un courant d'alimentation ou un éluant, et en outre au moins une sortie par laquelle on peut prélever dans le courant de liquide éventuellement en circulation des solutions du composé adsorbant le plus faiblement (raffinat) ou des solutions du composé adsorbant le plus fortement (extrait).

**4.** Procédé selon une ou plusieurs des revendications 2 à 3, **caractérisé en ce que** l'installation SMB comporte de 4 à 24 colonnes.

**5.** Procédé selon une ou plusieurs des revendications 2 à 4, **caractérisé en ce que** l'installation SMB contient des colonnes du type cylindrique à écoulement axial et équipées d'un dispositif pour compression dynamique.

**6.** Procédé selon une ou plusieurs des revendications 2 à 5, **caractérisé en ce que** l'installation SMB contient des colonnes ayant un diamètre de 5 à 1 500 mm.

**7.** Procédé selon une ou plusieurs des revendications 2 à 6, **caractérisé en ce que** l'installation SMB contient des colonnes ayant une longueur de 15 à 300 mm.

**8.** Procédé selon une ou plusieurs des revendications 3 à 7, **caractérisé en ce**, dans l'installation SMB, on produit un circuit fermé à courant de liquide en circulation.

**9.** Procédé selon une ou plusieurs des revendications 1 à 8, **caractérisé en ce que**, pour l'enrichissement en énantiomère, on part d'un mélange initial contenant les énantiomères du cis-8-benzyl-7,9-dioxo-2,8-diazabicyclo[4.3.0] nonane à un rapport molaire de 0,25 : 1 à 4 : 1.

**10.** Procédé selon une ou plusieurs des revendications 1 à 9, **caractérisé en ce que** le cis-8-benzyl-7,9-dioxo-2,8-diazabicyclo[4.3.0]-nonane est obtenu par hydrogénation du N-benzylimide de l'acide pyridine-2,3-dicarboxylique dans les cycles.

**11.** Procédé selon revendication 10, **caractérisé en ce que** l'enrichissement en énantiomère est réalisé en présence du trans-8-benzyl-7,9-dioxo-2,8-diazabicyclo[4.3.0]nonane ou de ses énantiomères et/ou d'autres produits d'accompagnement organiques provenant de l'hydrogénation du N-benzylimide de l'acide pyridine-2,3-dicarboxylique dans les cycles.

**12.** Procédé selon une ou plusieurs des revendications 1 à 11, **caractérisé en ce que**, pour l'enrichissement en énantiomère, on utilise des colonnes contenant en tant que phases stationnaire chirales des dérivés de polysaccharides, des polyacrylates chiraux ou des éthers en couronne chiraux, éventuellement appliqués sur une matière de support.

**13.** Procédé selon revendication 1 à 12, **caractérisé en ce que** l'on utilise des phases stationnaires chirales contenant du tris(3,5-diméthylphénylcarbamate) d'amylose, du tris-[(S)-$\alpha$-méthyl-benzylcarbamate] d'amylose, du tris(3,5-diméthylphénylcarbamate) de cellulose, du tris(4-méthylbenzoate) de cellulose ou du tris(4-chlorophénylcarbamate) de cellulose, appliqués sur gel de silice.

**14.** Procédé selon une ou plusieurs des revendications 12 à 13, **caractérisé en ce que** l'on utilise des phases stationnaires chirales contenant du tris(3,5-diméthylphénylcarbamate) d'amylose ou du tris(3,5-diméthylphénylcarbamate) de cellulose, appliquées sur gel de silice.

**15.** Procédé selon une ou plusieurs des revendications 1 à 14, **caractérisé en ce que** l'on utilise en tant que solvants des hydrocarbures aliphatiques en $C_6$-$C_{12}$, des éthers, des alcools aliphatiques en $C_1$-$C_6$, des nitriles ou des mélanges de ces solvants.

**16.** Procédé selon une ou plusieurs des revendications 1 à 15, **caractérisé en ce que** le solvant utilisé est l'acétonitrile.

**17.** Procédé selon une ou plusieurs des revendications 1 à 16, **caractérisé en ce que** l'on utilise un solvant à une teneur en humidité de 3 % en poids ou moins.

**18.** Procédé selon une ou plusieurs des revendications 2 à 17, **caractérisé en ce que**, à l'entrée du courant d'alimentation et de l'éluant, la pression est de 0,5 à 100 bar.

**19.** Procédé selon une ou plusieurs des revendications 1 à 18, **caractérisé en ce que** l'enrichissement en énantiomère est réalisé à une température de 0 à 80°C.

**20.** Procédé selon une ou plusieurs des revendications 1 à 19, **caractérisé en ce que** les énantiomères du cis-8-benzyl-7,9-dioxo-2,8-diazabicyclo[4.3.0]nonane sont obtenus à des puretés optiques de 70 % ee ou plus.

**21.** Procédé selon une ou plusieurs des revendications 1 à 20, **caractérisé en ce que** les énantiomères du cis-8-benzyl-7,9-dioxo-2,8-diazabicyclo[4.3.0]nonane sont obtenus à des puretés optiques de 90 % ee ou plus et une productivité supérieure à 0,2 kg/kg de phase stationnaire chirale et par jour.

**22.** Procédé selon une ou plusieurs des revendications 1 à 21, **caractérisé en ce que** l'énantiomère enrichi, non recherché, est soumis à racémisation.

**23.** Procédé selon revendication 22, **caractérisé en ce que** l'énantiomère enrichi, non recherché, est soumis à racémisation et recyclé à la chromatographie continue à contre-courant.

**24.** Procédé selon une ou plusieurs des revendications 22 à 23, **caractérisé en ce que** l'on obtient le (1S,6R)-8-benzyl-7,9-dioxo-2,8-diazabicyclo[4.3.0]nonane et **en ce que** l'on racémise l'énantiomère (1R,6S)-8-benzyl-7,9-dioxo-2,8-diazabicyclo[4.3.0]nonane avant de le recycler à la chromatographie continue à contre-courant.